## Europäisches Patentamt

## European Patent Office

## Office européen des brevets

(19)

(11) Veröffentlichungsnummer: **0 305 760 B1**

(12)

# EUROPÄISCHE PATENTSCHRIFT

(45) Veröffentlichungstag der Patentschrift: **27.10.93**

(51) Int. Cl.5: **C07K 3/22**, //C07K7/40

(21) Anmeldenummer: **88112661.9**

(22) Anmeldetag: **04.08.88**

(54) **Verfahren zur Isolierung basischer Proteine aus Proteingemischen.**

(30) Priorität: **11.08.87 DE 3726655**

(43) Veröffentlichungstag der Anmeldung:
**08.03.89 Patentblatt 89/10**

(45) Bekanntmachung des Hinweises auf die
Patenterteilung:
**27.10.93 Patentblatt 93/43**

(84) Benannte Vertragsstaaten:
**AT BE CH DE ES FR GB GR IT LI LU NL SE**

(56) Entgegenhaltungen:
**EP-A- 0 000 439**
**EP-A- 0 087 932**
**FR-A- 2 375 193**
**GB-A- 2 173 503**

**CHEMICAL ABSTRACTS, Band 108, 1988, Seite 336, Zusammenfassung Nr. 226915n, Columbus, Ohio, US; U. KRABIELL et al.: "Separation and purification of insulin from its B-components (proinsulin) on ion-exchange resins consisting of crosslinked acrylic acid polymers", & DD-A-247 684 (15-07-1987)**

(73) Patentinhaber: **HOECHST AKTIENGESELL-SCHAFT**

**D-65926 Frankfurt(DE)**

(72) Erfinder: **Dörschug, Michael, Dr.**
**Sonnenleite 20**
**D-4630 Bochum(DE)**
Erfinder: **Obermeier, Rainer, Dr.**
**Langenhainer Weg 14**
**D-6234 Hattersheim am Main(DE)**

EP 0 305 760 B1

Rank Xerox (UK) Business Services
(3.10/3.6/3.3.1)

EP 0 305 760 B1

**Beschreibung**

Bei der Herstellung von Humaninsulin mit gentechnischen Methoden erfolgt die Biosynthese des Insulins über ein Vorläufermolekül, das Proinsulin. In diesem ist die B-Kette durch das C-Peptid (= Connecting Peptide) mit der A-Kette verknüpft. Bei der gentechnischen Produktion mittels Escherichia coli erhält man mitunter zunächst ein Fusionsprotein, bei dem dem Proinsulin noch ein Fremdprotein, z.B. ß-Glactosidase, vorgeschaltet ist. Dieses Fremdprotein muß vor der weiteren Aufarbeitung zunächst abgespalten werden, z.B. durch die Behandlung mit Halogencyan (vgl. DE-OS 34 40 988). Nach der Halogencyanspaltung werden Cysteinreste durch Sulfitolyse (Behandlung mit Natriumsulfit und Natriumtetrathionat) in ihre S-Sulfonatform überführt. Das Molekül kann aus dieser Form durch reduktive Rückfaltung (z.B. durch Behandlung mit Mercaptoethanol in basischer Lösung) unter korrekter Ausbildung seiner Disulfidbrücken in seine native Raumstruktur überführt werden. Das Proinsulin bzw. Präproinsulin (= Derivat des Proinsulins; die Vorsilbe "Prä" bezieht sich auf eine oder mehrere zusätzliche Aminosäuren am N-Terminus des Proinsulins) wird durch enzymatische Spaltung (z.B. mit Trypsin) in ein Spaltungsgemisch überführt, das einen Insulinvorläufer, das Insulin-Arg-B31-B32, und das C-Peptid enthält. Neben diesen entstehen noch einige Nebenprodukte, wie z.B. Insulin-Des-Thr-B30, Insulin-Arg-B31 sowie unvollständig gespaltene Intermediate.

Die genannten Insulinderivate und Verbindungen, die durch die chemische Behandlung des Ausgangsmaterials in Vorstufen entstehen, müssen voneinander getrennt werden. Besonders schwierig ist es hierbei, Insulinderivate basischen Charakters abzutrennen, die Derivatisierungen an Aminosäuren aufweisen, die nach Ausbildung der Tertiärstruktur im Inneren des Moleküls liegen.

Dies zeigt sich in einem Vergleichsversuch (s. Teil B): Das Anionenaustauscherverfahren, das in der DE-PS 26 29 568 beschrieben und für die Reinigung von Insulin entwickelt worden ist, weist die Besonderheit auf, daß zur Vermeidung von Proteinaggregationen der Elutionsflüssigkeit ein nichtionisches Tensid zugegeben wird. Dieses Verfahren, das zur Reinigung von Insulinen gut geeignet ist, führt beim Versuch der Trennung und Isolierung von basischen Proteinen, die z.B. durch tryptische Spaltung von Proinsulin gentechnischer Herkunft erhalten werden, zu einer völlig unzureichenden Trennung. Dies wird durch das entsprechende Elutionsprofil (s. Teil B) verdeutlicht; man erkennt, daß die Peaks für die einzelnen Peptide ineinander übergehen.

Es sind auch bereits Verfahren bekannt, mit denen die Auftrennung der genannten Proinsulin-Spaltungsprodukte erreicht werden soll.
Steiner et al. berichten im "Journ. of Biol. Chem.", 246, pp. 1365-1374 (1971) über ein Verfahren zur Isolierung von C-Peptid aus einem Proinsulinspaltungsgemisch. Bei diesem Verfahren (Ausbeuten werden nicht angegeben) wird über einen schwach sauren, Carboxymethylgruppen-haltigen (CM) Kationenaustauscher auf Cellulosebasis chromatographiert. Der Beladungs- und Elutionslösung werden hierbei 7 Mol/l Harnstoff zugesetzt, um eine Aggregation der Proteine zu vermeiden. Die Gegenwart derartig hoher Harnstoffkonzentrationen ist nachteilig, weil sie zur Derivatisierung von Proteinen, vor allem zur Carbamoylierung freier Aminogruppen führt. Markussen et al. beschreiben in "Protein Engineering " Vol. 1 No. 3, pp. 205-213 (1987) ein Anionenaustauscherchromatographie-Verfahren an Diethyl-2-hydroxypropylaminoethyl-haltigen (QAE) Anionenaustauschern auf einer Matrix aus dreidimensional vernetztem Polysaccharid-Netzwerk.
Bei diesem Verfahren - für die Anionenaustauscherchromatographie werden keine Ausbeuten angegeben - wird zur Hemmung von Proteinaggregationen in 60 %iger Ethanollösung gearbeitet. Ein Nachteil derartig konzentrierter Ethanollösungen besteht in den nötigen Sicherheitsvorkehrungen, die insbesondere beim Arbeiten mit konzentrierten organischen Lösungsmitteln in technischem Maßstab nötig sind. Andererseits treten, wie sich beim Nacharbeiten zeigte, bei den genannten Alkoholkonzentrationen Denaturierungen der Proteine auf.

In dem Bestreben, ein besseres Trenn- und Isolierverfahren für basische Proteine aus - solche basischen Proteine enthaltenden - Proteingemischen bereitzustellen, wurde überraschenderweise gefunden, daß sich dieses Ziel durch Chromatographie der Proteingemische an einem stark sauren Kationenaustauscher und Elution mittels wäßrigem Alkanol mit nur einer relativ geringen Menge Alkanol erreichen läßt. Es ist besonders überraschend, daß sich damit gegenüber dem Stand der Technik sowohl die Trennleistung verbessern als auch die zusätzlichen Derivatisierungen der Proteine während der Trennung weitgehend vermeiden lassen. Bemerkenswerterweise können sogar Derivate abgetrennt werden, die - bei der Vorbehandlung des Proinsulins entstandene - Derivatisierungen im Inneren der in der Tertiärstruktur vorliegenden Proteine aufweisen. Aggregationen der zu trennenden Proteine werden nicht beobachtet. Die Wiederverwendbarkeit der Austauscher bereitet keine Schwierigkeiten.

2

EP 0 305 760 B1

Erfindungsgegenstand ist demzufolge ein Verfahren zur Isolierung basischer Proteine aus Proteingemischen, welche solche basischen Proteine enthalten und welche erhalten wurden durch enzymatische Spaltung von Proinsulin und/oder dessen Derivaten natürlicher, semisynthetischer oder gentechnischer Herkunft, durch Beladung eines Ionenaustauschers mit dem Proteingemisch und Elution,

welches dadurch gekennzeichnet ist, daß man als Ionenaustauscher einen stark sauren Kationenaustauscher verwendet und daß man die Elution mittels eines $H_2O/C_1$-$C_4$ Alkanolgemisches, das ca. 10 bis 50 Vol.-%, vorzugsweise ca. 20 bis 40 Vol.-%, insbesondere ca. 30 Vol.-% Alkanol enthält, durchführt. Mit dem erfindungsgemäßen Verfahren können aus Spaltungsgemischen beliebiger Proinsuline, bzw. von deren Derivaten, wie z.B. von Affenpräproinsulin, die basischen Proteine mit hohen Ausbeuten isoliert werden. Als Ionenaustauscher kommen im Prinzip alle stark sauren Kationenaustauscher in Frage. Bevorzugt sind stark saure Kationenaustauscher, deren funktionelle Gruppe die Sulfogruppe, insbesondere die Sulfopropylgruppe -$CH_2$-$CH_2$-$CH_2$-$SO_3H$, ist, z.B. auf einer Matrix aus hydrophilen Hydroxygruppen-haltigen Vinylpolymeren (z.B. [R]Fractogel TSK, Hersteller Fa. Merck, Darmstadt), Acryl-Copolymeren (z.B. SP [R]Trisacryl M, Hersteller Réactifs IBF, Villeneuve- la-Garenne, Frankreich) oder Gelen mit einem Anteil an vernetzter Agarose (z.B. S-[R]Sepharose, Hersteller Fa. Pharmacia, Upsala, Schweden); besonders bevorzugt ist S-Sepharose.

Vor der Beladung des stark sauren Kationenaustauschers mit dem Spaltungsgemisch sollte derselbe mit einer Pufferlösung frisch equilibriert werden. Diese Pufferlösung besteht vorzugsweise aus einem Wasser/$C_1$-$C_4$-Alkanolgemisch, mit einem Alkanolgehalt von vorzugsweise ca. 10 bis 50 Vol.-%, besonders bevorzugt von ca. 20 bis 40 Vol.-%, insbesondere von ca. 30 Vol.-%. Bevorzugte Alkanole sind Ethanol und Isopropanol, insbesondere Isopropanol. Weitere Zusatzstoffe, die der Pufferlösung zugesetzt werden können, sind z.B. Salz, vorzugsweise physiologisch verträgliches Mineralsalz, eine oder mehrere beliebige organische Säuren, vorzugsweise Milchsäure, eine Base, vorzugsweise NaOH, und/oder Konservierungsstoffe. Der bevorzugte pH-Wert der Pufferlösung liegt zwischen ca. 2,5 und 5,5, besonders bevorzugt zwischen ca. 3,5 und 4,0.

Die Beladung des stark sauren Kationenaustauschers kann erfolgen, indem das Spaltungsgemisch in einer Pufferlösung -vorzugsweise mit der zuvor beschriebenen Zusammensetzung und dem zuvor beschriebenen pH-Wert - gelöst wird und die so erhaltene Lösung mit dem stark sauren Kationenaustauscher in Kontakt gebracht wird.

Die Elutionslösung, die im Prinzip eine ähnliche Zusammensetzung wie die zuvor beschriebene Pufferlösung haben kann, hat vorzugsweise einen pH-Wert von 3,5 bis 4,0. Besonders geeignet ist ein Elutionsverfahren, bei dem die Elutionslösung einen zeitlichen Salz-Konzentrationsgradienten mit vorzugsweise linearem Verlauf aufweist. Dieser Konzentrationsgradient kann z.B. angelegt werden, indem zu Beginn der Elution eine geringe Salzkonzentration (im Grenzfall gegen O) in der Elutionslösung vorliegt und indem die Salzkonzentration während des Elutionsvorgangs gesteigert wird. Auf diese Weise kann eine besonders wirksame Trennung des Proteingemisches erreicht werden. Ein bevorzugter Salz-Konzentrationsgradient variiert von nahe O Mol Salz/l (zu Anfang der Elution) bis ca. 1 Mol Salz/1 (zu Ende der Elution), besonders bevorzugt von ca. 0,15 (zu Anfang der Elution) bis ca. 0,35 Mol/l (zu Ende der Elution). Für den Salzzusatz kommen viele organische und anorganische Salze in Frage. Bevorzugt sind physiologisch verträgliche Salze wie Ammonium- und Alkalisalze, besonders bevorzugt Natriumsalze, insbesondere Natriumchlorid.

Das erfindungsgemäße Trennverfahren kann auf unterschiedliche Weise durchgeführt werden. Zu bevorzugen ist die Durchführung im Säulenverfahren oder im Batchverfahren. Die Temperatur, die bei der Ionenaustauscherchromatographie vorzugsweise konstant zu halten ist, kann in einem weiten Bereich variiert werden. Vorzuziehen ist ein Temperaturintervall von ca. -10 °C bis ca. 50 °C, insbesondere von ca. 15 bis ca. 25 °C.

Durch das nachfolgende Ausführungsbeispiel (A) soll die Erfindung näher erläutert werden. Durch den daran anschließenden Vergleich (B) soll die Überlegenheit des erfindungsgemäßen Verfahrens gegenüber einem Verfahren zur Insulinreinigung gemäß dem Stand der Technik (DE-PS 26 29 568) gezeigt werden.

**A) Ausführungsbeispiel** (erfindungsgemäß)

6 l S-Sepharose werden in einer wäßrigen Pufferlösung, die 50 mmol/l Milchsäure, 30 % Isopropanol und 1 Mol/l Natriumchlorid enthält und deren pH-Wert bei ca. 3,5 liegt, aufgeschlämmt und ca. 80 cm hoch in eine Säule mit 10 cm Durchmesser gefüllt. Die Säule wird mit 10 l wäßriger Startpufferlösung (50 mmol/l Milchsäure, 30 % Isopropanol, 0,15 Mol/l Natriumchlorid, pH ca. 3,5) äquilibriert. 15 g kristallisiertes Spaltungsgemisch, das 6,2 g Insulin-Arg-B31-32, 3 g Insulin-Arg-B31 und Insulin-Des-Thr-B30 sowie unbekannte Spaltungsintermediate sowie Derivate enthält, entstanden aus tryptischer Spaltung von Präprohumaninsulin, werden in 3 l Startpuffer gelöst und auf die Säule aufgetragen. Anschließend wird mit einer wäßrigen Lösung, enthaltend 50 mmol/l Milchsäure und 30 % Isopropanol bei einem pH-Wert von 3,5 (eingestellt mit NaOH) und einem Gradienten von 0,15 Mol./l bis 0,35 Mol/l Natriumchlorid eluiert. Die Elutionslösung hat ein Volumen von 2 x 20 1. Die Durchflußgeschwindigkeit beträgt 1,5 l/Stunde. Fraktionen, deren Insulin-Arg-B31-32-Gehalt laut HPLC (High Pressure Liquid Chromatography) über 90% ist, werden gesammelt, mit $H_2O$ im Verhältnis 1:1 verdünnt und durch Zugabe von 10 ml 10 %iger $ZnCl_2$-Lösung/l Lösung und Einstellung auf pH 6,8 gefällt. Die Ausbeute beträgt 5 g Insulin-Arg-B31-32, was einer Stufenausbeute, bezogen auf Insulin-Arg-B31-32, von 80 % entspricht.

**B) Vergleich**

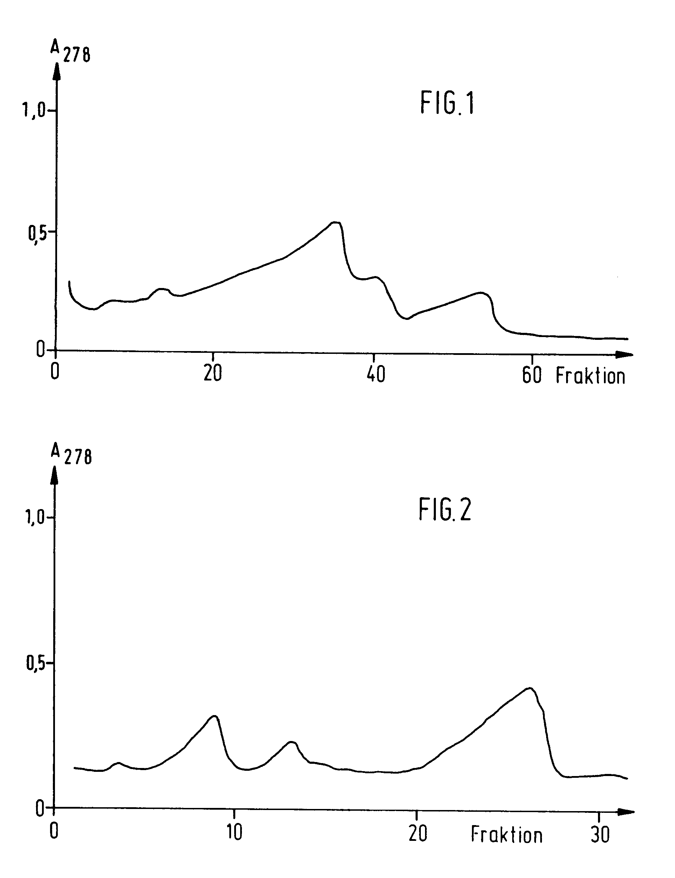

Vergleichsbeispiel I wurde gemäß DE-PS 26 29 568 und Beispiel II erfindungsgemäß durchgeführt. In Tabelle 1 sind die Verfahrensparameter und die erzielten Ergebnisse gegenübergestellt. Figur 1 zeigt das während der Elution aufgenommene Elutionsprofil des Vergleichsbeispiels I, Figur 2 dasjenige des erfindungsgemäßen Beispiels II. Es wurde die Absorption A im UV-Bereich bei einer Wellenlänge von 278 nm ($A_{278}$) gegen die Elutionszeit bzw. die Fraktions- Nrn. aufgetragen. Die Elutionsprofile zeigen, daß das erfindungsgemäße Verfahren zu einer besseren Trennung der basischen Proteine führt als das nach der DE-PS 26 29 568 durchgeführte Verfahren. Dementsprechend wird bei dem erfindungsgemäßen Verfahren auch eine höhere Produktausbeute und eine höhere Produktreinheit erzielt (s. Tabelle 1)

**Tabelle 1**

Vergleich Ionenaustauscherchromatographie eines Proteingemisches, erhalten durch Behandlung von Proinsulin (Affenproinsulin, gentechnisch mittels E.coli hergestellt) mit Trypsin

| | I Vergleichsbeispiel gemäß DE-PS 26 29 568 | II Erfindungsgemäß |
|---|---|---|
| Austauscher: | (R) TSK-DEAE-Anionenaustauscher (funktionelle Gruppen: Diethylaminoethyl, Matrix: hydroxylgruppenhaltiges Vinylpolymeres: Hersteller: Fa. Merck, Darmstadt) | S-(R) Sepharose-Kationenaustauscher (funktionelle Gruppen: Sulfopropylgruppen; Matrix: hydroxylgruppenhaltiges Gel mit einer Anteil an vernetzter Agarose; Hersteller: Fa. Pharmacia, Schweden) |
| Puffer, Gradient: | Tris(hydroxymethyl)aminomethan/HCl pH 9, 0.1-0.25 M NaCl | Milchsäure, pH3.5, 0.15-0.35 M NaCl |
| Dissoziierendes Agens: | 0.1 % Genapol(R) SE 100 (Fettalkoholpolyglykoläther, Hersteller: Fa. Hoechst AG, Frankfurt) | 30 % Isopropanol |
| Beladung: | 1 g/l | 3 g/l |
| Stufenausbeute: | ca. 60 % | ca. 80 % |
| Reinheit: | 80 – 85 % | 90 – 95 % |
| Trennung: | keine vollständige Trennung zw. Insulin-Arg-B31-32, Insulin-Arg-B31 und basischen Verunreinigungen | Basislinientrennung zwischen Insulin-Arg-B31-32, Insulin-Arg-B31 und basischen Verunreinigungen |

**Patentansprüche**

1. Verfahren zur Isolierung basischer Proteine aus Proteingemischen, welche solche basischen Proteine enthalten und welche erhalten wurden durch enzymatische Spaltung von Proinsulin und/oder dessen Derivaten natürlicher, semisynthetischer oder gentechnischer Herkunft, durch Beladung eines Ionenaustauschers mit dem Proteingemisch und Elution, dadurch gekennzeichnet, daß man als Ionenaustauscher einen stark sauren Kationenaustauscher verwendet und daß man die Elution mittels eines $H_2O/C_1$-$C_4$-Alkanol-Gemisches, das ca. 10 bis 50 %, vorzugsweise ca. 20 bis 40 %, insbesondere ca. 30 % Alkanol enthält, durchführt.

2. Verfahren nach Anspruch 1, dadurch gekennzeichnet, daß man als stark sauren Kationenaustauscher einen solchen mit Sulfogruppen, insbesondere mit Sulfopropylgruppen verwendet.

3. Verfahren nach Anspruch 1 oder 2, dadurch gekennzeichnet, daß man die Beladung des stark sauren Kationenaustauschers bei einem pH-Wert zwischen ca. 2,5 und 5,5, vorzugsweise zwischen ca. 3,5 und 4,0, durchführt.

4. Verfahren nach einem oder mehreren der Ansprüche 1 bis 3, dadurch gekennzeichnet, daß das zur Elution benutzte $H_2O/C_1$-$C_4$-Alkanol-Gemisch als $C_1$-$C_4$-Alkanol Ethanol oder Isopropanol, insbesondere Isopropanol, enthält.

5. Verfahren nach einem oder mehreren der Ansprüche 1 bis 4, dadurch gekennzeichnet, daß man den pH-Wert der Elutionslösung auf ca. 3,5 bis 4,0 einstellt.

6. Verfahren nach einem oder mehreren der Ansprüche 1 bis 5, dadurch gekennzeichnet, daß die Beladungs- und Elutionslösung eine Puffersubstanz, bevorzugt auf Basis einer organischen Säure, insbesondere Milchsäure, enthält.

7. Verfahren nach einem oder mehreren der Ansprüche 1 bis 6, dadurch gekennzeichnet, daß mit einem Ammonium- oder Alkalisalzgradienten zwischen ca. 0 bis 1 Mol/l, insbesondere mit ca. 0,15 bis 0,35 Mol/l, eluiert wird.

**Claims**

1. A process for the isolation of basic proteins from a protein mixture which contains such basic proteins and which has been obtained by enzymatic cleavage of proinsulin and/or one of its derivatives of natural, semisynthetic or genetic engineering origin by loading an ion exchanger with the protein mixture and elution, which comprises using a strongly acid cation exchanger as the ion exchanger and carrying out the elution by means of an $H_2O/C_1$-$C_4$-alkanol mixture which contains about 10 to 50%, preferably about 20 to 40% and in particular about 30%, of alkanol.

2. The process as claimed in claim 1, wherein the strongly acid cation exchanger used is one with sulfo groups, in particular with sulfopropyl groups.

3. The process as claimed in claim 1 or 2, wherein the loading of the strongly acid cation exchanger is carried out at a pH between about 2.5 and 5.5, preferably between about 3.5 and 4.0.

4. The process as claimed in one or more of claims 1 to 3, wherein the $H_2O/C_1$-$C_4$-alkanol mixture used for the elution contains ethanol or isopropanol, in particular isopropanol, as the $C_1$-$C_4$-alkanol.

5. The process as claimed in one or more of claims 1 to 4, wherein the pH of the elution solution is brought to about 3.5 to 4.0.

6. The process as claimed in one or more of claims 1 to 5, wherein the loading and elution solution contains a buffer substance, preferably based on an organic acid, in particular lactic acid.

7. The process as claimed in one or more of claims 1 to 6, wherein the elution is carried out with an ammonium or alkali metal salt gradient of between about 0 to 1 mol/l, in particular with about 0.15 to 0.35 mol/l.

**Revendications**

1. Procédé pour l'isolement de protéines basiques à partir de mélanges de protéines qui contiennent de telles protéines basiques et qui ont été obtenus par coupure enzymatique de proinsuline et/ou de ses dérivés d'origine naturelle, semi-synthétique ou produits par génie génétique, par charge d'un échangeur d'ions avec le mélange de protéines et élution, caractérisé en ce que, en tant qu'échangeur d'ions, on utilise un échangeur de cations fortement acide, et en ce que l'on effectue l'élution au moyen d'un mélange d'eau et d'un alcanol en $C_1$-$C_4$, qui contient environ 10 à 50 %, de préférence environ 20 à 40 %, en particulier environ 30 % d'alcanol.

2. Procédé selon la revendication 1, caractérisé en ce que l'on utilise, en tant qu'échangeur de cations fortement acide, un tel échangeur comportant des groupes sulfo, en particulier comportant des groupes sulfopropyle.

3. Procédé selon la revendication 1 ou 2, caractérisé en ce que l'on effectue la charge de l'échangeur de cations fortement acide à un pH entre environ 2,5 et 5,5, de préférence entre environ 3,5 et 4,0.

4. Procédé selon une ou plusieurs des revendications 1 à 3, caractérisé en ce que le mélange d'eau et d'alcanol en $C_1$-$C_4$, utilisé pour l'élution, contient, en tant qu'alcanol en $C_1$-$C_4$, de l'éthanol ou de l'isopropanol, en particulier de l'isopropanol.

5. Procédé selon une ou plusieurs des revendications 1 à 4, caractérisé en ce que le pH de la solution d'élution est ajusté à environ 3,5 à 4,0.

6. Procédé selon une ou plusieurs des revendications 1 à 5, caractérisé en ce que la solution de charge d'élution contient une substance tampon, de préférence à base d'un acide organique, en particulier d'acide lactique.

7. Procédé selon une ou plusieurs des revendications 1 à 6, caractérisé en ce que l'on effectue l'élution avec un gradient de sels alcalins ou d'ammonium compris entre environ 0 et 1 mole/l, en particulier avec environ 0,15 à 0,35 mole/l.

FIG.1

FIG.2